# EUROPÄISCHE PATENTSCHRIFT

(11) **EP 2 370 006 B1**
(45) Veröffentlichungstag und Bekanntmachung des Hinweises auf die Patenterteilung: **21.12.2016**
(21) Anmeldenummer: 09759996.3
(22) Anmeldetag: 05.11.2009
(51) Int. Cl.: A61B 17/3207, A61B 17/00

(54) **KATHETER ZUM ANSAUGEN, FRAGMENTIEREN UND HINAUSBEFÖRDERN VON ENTFERNBAREM MATERIAL AUS BLUTGEFÄSSEN**
CATHETER FOR ASPIRATING, FRAGMENTING AND REMOVING REMOVABLE MATERIAL FROM BLOOD VESSELS
CATHÉTER POUR L'ASPIRATION, LA FRAGMENTATION ET L'ÉVACUATION DE MATÉRIAU À ÉLIMINER DE VAISSEAUX SANGUINS

(30) Priorität: 27.11.2008 CH 18502008
(43) Veröffentlichungstag der Anmeldung: 05.10.2011
(73) Patentinhaber: Straub Medical AG, 7323 Wangs (CH)
(72) Erfinder: STRAUB, Immanuel, CH-7323 Wangs (CH)
(74) Vertreter: Patentbüro Paul Rosenich AG
(86) Internationale Anmeldenummer: PCT/IB2009/054909
(87) Internationale Veröffentlichungsnummer: WO 2010/061308

(56) Entgegenhaltungen:
- WO-A1-00/47116
- WO-A1-96/29941
- WO-A2-2005/084562
- US-A- 5 569 178
- US-A- 5 833 704
- US-A1- 2002 010 487

## Beschreibung

Die Erfindung bezieht sich auf Katheter zum Ansaugen, Fragmentieren und Hinausfördern von entfernbarem Material aus Hohlkörpern, insbesondere von Thromben und Embolien aus Blutgefässen, mit einem über einen Führungsdraht unabhängig von diesem axial verschiebbaren, am distalen Ende des Katheters angeordneten Arbeitskopf, welcher wenigstens eine seitliche Öffnung aufweist, wobei der Katheter eine mittels eines vom Arbeitskopf entfernten Drehantriebes einer Antriebseinheit mit einer Drehzahl in Rotation versetzbare flexible Förderschraube mit einem distalen und einem proximalen Teil aufweist, sowie mit einem die Förderschraube umgebenden, mit dem Arbeitskopf verbundenen flexiblen Schlauch zur Abfuhr des Materials bzw. der abgelösten Thromben und Emboliefragmente und einem Schneidwerkzeug, wobei die Förderschraube als mit der Öffnung des Arbeitskopfes zusammenwirkendes, scherendes Schneidwerkzeug ausgebildet ist, um zwischen den peripheren Rändern der Förderschraube und Rändern der Öffnungen die eindringenden Materialien bzw. angesaugten und/oder abgelösten Thromben und Embolien zu zerkleinern.

Aus der WO 2005/084562 A2 der Anmelderin sind Katheter gemäß dem Oberbegriff bekannt, die insbesondere zum Entfernen von frischen Blutgerinnseln aus Blutgefässen eingesetzt werden, um zu verhindern, dass sich diese an Engpässen stauen und zum Verschluss der Blutgefässe (sog. Embolien) führen.

Die WO 96/29941 A1 zeigt einen Rotationskatheter für die Atherektomie, dessen Arbeitskopf aus einem stillstehenden, mit einem Schlauch verbundenen Stator und einem Rotor besteht. Der Rotor ist gegenüber dem Stator mittels einer hochtourigen Förder/Antriebsschraube verdrehbar. Sowohl der Stator als auch der Rotor weisen an ihrem Umfang in Übereinstimmung bringbare Fenster auf. Durch Scherung zwischen einer Schneide am Rotor und einer Gegenschneide an den Öffnungen des Stators wird eine Zerkleinerung der in die Öffnungen hineinragenden, bzw. eingesaugten Teile bewirkt. Der Rotor kann den Stator aussen umgeben ("Aussenläufer") oder im Inneren des Stators angeordnet sein ("Innenläufer"). Dieser Rotationskatheter wird vor allem dort eingesetzt, wo sich im Laufe der Zeit relativ harte und eher spröde Beläge an der Innenwand von Blutgefässen wie Venen und dgl. gebildet haben. Zum Entfernen von frischen Blutgerinnseln (wie z.B. Thromben) aus Blutgefässen ist die Bauart gemäss WO 2005/084562 A2 jedoch besser geeignet.

Die zu entfernenden Blutgerinnsel sind oft sehr faserig und zäh und können nur mühsam, mit grossem Zeit- und Geräte-Aufwand teilweise oder gar nicht entfernt werden. Mitunter verstopft das Verschlussmaterial die Gänge der Förderschraube, sodass gar kein Material mehr gefördert werden kann. Bei einem Blockieren der Förderschraube kann diese beschädigt und der Katheter somit unbrauchbar werden.

Aus der US 5,569,178 A ist ein Katheter bekannt, bei dem das distale Ende der Förderschraube in einem Axial-Drucklager gelagert ist, welches in einer entsprechenden Ausnehmung vorgesehen ist.

Der Erfindung liegt daher die Aufgabe zugrunde, einen Katheter zu schaffen, der auch unter schwierigen Verhältnissen ein rasches, störungsfreies und vollständiges Entfernen von Thromben und Embolien ermöglicht.

Gemäss der Erfindung wird dieses dadurch erreicht, dass zwischen dem distalen Ende der Förderschraube und dem Arbeitskopf ein Axial-Drucklager angeordnet ist, das als beim Arbeitsgang durch die Förderschraube in Rotation versetzbare Scheibe ausgebildet ist.

Ein solches Axial-Drucklager nimmt die axialen Reaktionskräfte auf, welche an der Förderschraube insbesondere dann entstehen, wenn Material vom distalen zum proximalen Ende des Katheters gefördert wird. Diese Lösung ist konstruktiv einfach und besonders. Der Aussendurchmesser der Scheibe entspricht etwa dem Aussendurchmesser der Förderschraube und der Innendurchmesser der Scheibe entspricht etwa dem Aussendurchmesser des Führungsdrahtes.

Beim Antrieb der Förderschraube tritt zwischen der Förderschraube und der Scheibe eine Relativ-Verdrehung auf, wodurch sich diese mit Axialdruck nach distal verlängert. Damit dabei keine blutschädigende Reibungswärme entsteht, besteht die Scheibe vorteilhaft aus einem Werkstoff mit geringem Reibungskoeffizienten, vorzugsweise aus Kunststoff. Um Abrieb möglichst zu vermeiden, sollte ein relativ harter Werkstoff gewählt werden.

Die Förderschraube ist zweckmässigerweise unter axialem Druck vorspannbar. Die axialen Vorspannkräfte werden dabei durch das Axial-Drucklager aufgenommen. Die Förderschraube ist somit vom Beginn der Operation an in einem definierten Zustand. Vibrationen können dabei weitgehend reduziert oder verhindert werden.

Je nach Art des Verschlussmaterials und Lage der zu öffnenden Stelle des Blutgefässes können die Anforderungen an den Katheter unterschiedlich sein. Der Vorspanndruck der Förderschraube ist daher vorteilhaft einstellbar. Das Einstellen des Vorspanndruckes erfolgt vorzugsweise ab Werk.

Der Vorspanndruck der Förderschraube ist zweckmässigerweise so eingestellt, dass das Axial-Drucklager etwa mit der halben Drehzahl der Förderschraube mitrotiert. Somit besteht zwischen dem Axial-Drucklager und dem Arbeitskopf einerseits und der Förderschraube andererseits je ein Relativ-Drehzahlunterschied in Grössenordnung von etwa der halben Drehzahl der Förderwendel. Dadurch kann einerseits die Wärmeentwicklung reduziert und andererseits ein Abrieb der sich relativ zueinander bewegenden Bauteile minimiert oder eliminiert werden.

Um ein Verstopfen der Öffnungen des Arbeitskopfes durch das zu entfernende Verschlussmaterial möglichst zu vermeiden, ist vorteilhaft die wenigstens eine seitliche Öffnung des Arbeitskopfes als kreisrundes Loch mit jeweils einem Kreisumfang ausgebildet. Die Kreisform des Loches ergibt einen im Verhältnis zur Grösse günstigen Querschnitt und ist auch strömungstechnisch günstig.

Eine zweckmässige Lösung besteht darin, dass die wenigstens eine seitliche Öffnung des Arbeitskopfes als jeweils wenigstens zwei, axial hintereinander angeordnete Löcher ausgebildet ist. Durch eine Mehrzahl von Löchern kann der Querschnitt der Öffnung direkt vervielfacht werden.

Bei der Anordnung von mehreren, axial hintereinander angeordneten Löchern überschneiden sich die Kreisumfänge der Löcher vorteilhaft. Die einzelnen Löcher bilden somit zusammen eine gemeinsame Öffnung mit mehreren verschiedenen Schneidkanten.

Anstelle der sich überschneidenden Anordnung der Löcher oder auch zusätzlich zu einer solchen Anordnung können die Löcher auch zweckmässig durch einen in axialer Richtung verlaufenden, im wesentlichen zentrisch zu den Löchern angeordneten Schlitz miteinander verbunden sein. Ein solcher Schlitz vergrössert nicht nur den Querschnitt der Öffnung, sondern er bildet auch zusätzliche Schneiden zum Zerkleinern des zu entfernenden Materials.

Besonders gefährdet bezüglich eines Verklemmens des zu entfernenden Verschlussmaterials zwischen der Förderschraube und den seitlichen Öffnungen des Arbeitskopfes ist der rückwärtige, antriebsseitige Bereich der Öffnungen. Es ist daher vorteilhaft, dass sich der Schlitz in Richtung zum proximalen Ende des Katheters über die Löcher hinaus erstreckt. In diesem gefährdeten Bereich entstehen somit zusätzliche, in axialer Richtung angeordnete Schneidkanten.

Am Arbeitskopf sind zweckmässigerweise jeweils zwei einander etwa diametral gegenüberliegend angeordnete Öffnungen ausgebildet. Durch zwei einander diametral gegenüberliegende Öffnungen wird nicht nur der Durchtrittsquerschnitt vergrössert, sondern es werden auch die Anzahl der Schneidkanten und somit die Zahl der Schneidvorgänge pro Umdrehung des Katheters verdoppelt.

Um gute Schneidvorgänge zur Zerkleinerung des zu entfernenden Materials im Katheter zu ermöglichen, ist der distale Teil der Förderschraube im Bereich des Arbeitskopfes im Aussendurchmesser gegenüber dem Innendurchmesser des vorzugsweise zylindrischen Arbeitskopfes vorteilhaft passgenau ausgebildet, so dass der Aussendurchmesser der Förderschraube zum Innendurchmesser der Innenmantelfläche des Arbeitskopfes ein nur minimales Durchmesserspiel aufweist. Dabei wird verhindert, dass Teile des zu entfernenden Materials in den Spalt zwischen der Förderschraube und dem Arbeitskopf gelangen und es zu einem gegenseitigen Verklemmen zwischen dem Arbeitskopf und der Förderschraube kommen kann.

Durch das Zusammenwirken der rotierenden Förderschraube mit den Öffnungen der Arbeitskopfes entstehen Schneid-, bzw. Schervorgänge am zu entfernenden, in den Katheter eingesaugten Material. Damit diese Schneidvorgänge optimal verlaufen, sind die Kanten an der Aussenseite der Förderschraube im Bereich der

Öffnung des Arbeitskopfes zweckmässigerweise scharfkantig ausgebildet. Dieses ermöglicht ein gutes und sauberes Abscheren der zu entfernenden, meistens sehr zähen Thromben und Embolien.

Der Arbeitskopf ist zweckmässigerweise in Richtung seines distalen Endes verjüngt ausgebildet. Dadurch wird erreicht, dass der Katheter auch in engen Krümmungsradien der Blutgefässe ohne grösseren Widerstand gleitend vorgeschoben werden kann. Ausserdem kann er sich an der Gefässwandung bzw. an Vorsprüngen der Blutgefässe nicht einhaken.

Die Kanten der seitlichen Öffnungen sind auf der Innenseite vorteilhaft wenigstens bereichsweise scharfkantig ausgebildet. Dadurch wird, zusammen mit der Peripherie der Förderschraube, ein sauberer Schervorgang zum Fragmentieren der Thromben oder Embolien ermöglicht. Die Öffnungen im Arbeitskopf sind so konstruiert, dass durch die mit hoher Drehzahl rotierende Förderschraube Thromben und Embolien, welche angesaugt werden, an den inneren scharfen Kanten der Öffnungen und an der Peripherie der Förderschraube in kleine Partikel fragmentiert werden. Diese im Blut schwebenden Partikel werden durch den bestehenden Unterdruck sowie die Wirkung der Förderschraube in Richtung des Drehantriebes gefördert.

Die Kanten der seitlichen Öffnung sind auf der Mantelseite des Arbeitskopfes zweckmässigerweise wenigstens bereichsweise abgerundet. Dieses ermöglicht eine praktisch wirbelfreie Strömung der zu entfernenden Ablagerungen sowie anderer abgesaugter Körperflüssigkeiten (z.B. Blut) im Bereich des Arbeitskopfes.

Weitere Ausbildungen der Erfindung sind in den Figuren und in den abhängigen Patentansprüchen angegeben.

Die Bezugszeichenliste ist Bestandteil der Offenbarung.

Anhand der Figuren wird die Erfindung symbolisch und beispielhaft näher erläutert.

Die Figuren werden zusammenhängend und übergreifend beschrieben. Gleiche Bezugszeichen bedeuten gleiche Bauteile; Bezugszeichen mit unterschiedlichen Indizes geben funktionsgleiche oder ähnliche Bauteile an.

Es zeigen dabei:
- Fig. 1: einen erfindungsgemässen Katheter, in perspektivischer Darstellung,
- Fig. 2: den Endbereich des Katheters gemäss Fig. 1,
- Fig. 3: den Katheter gemäss Fig. 1 und 2, im Längsschnitt,
- Fig. 4: eine Seitenansicht des Arbeitskopfes eines Katheters, gemäss Fig. 1 bis Fig. 3,
- Fig. 5: eine perspektivische Darstellung des Arbeitskopfes gemäss Fig. 4
- Fig. 6: eine perspektivische Darstellung des Axial-Drucklagers und
- Fig. 7: eine perspektivische Darstellung der Antriebs- und Förderschraube.

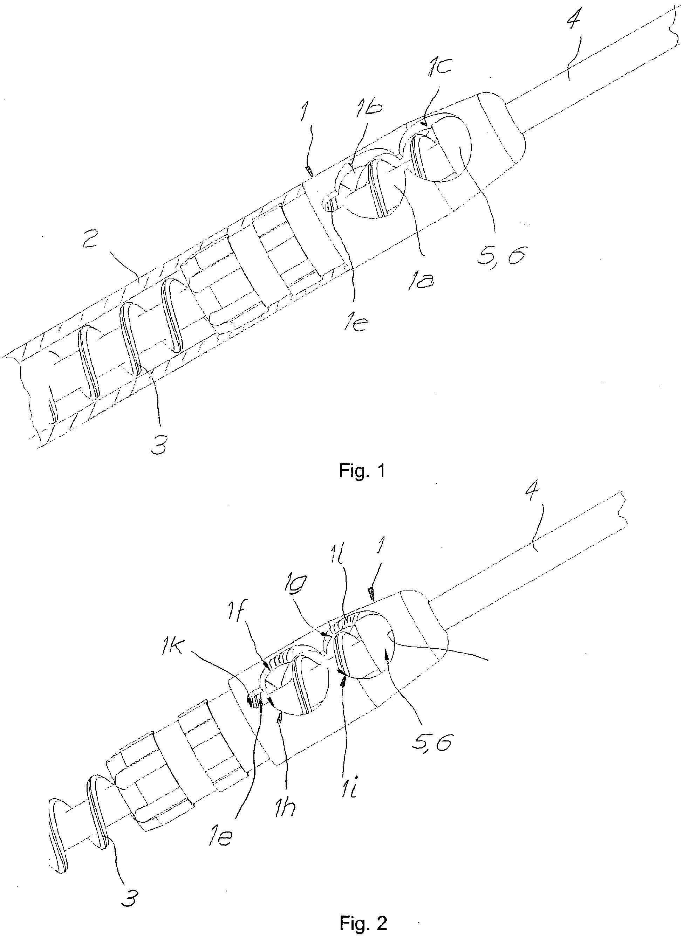

Der aus Fig. 1 bis Fig. 3 ersichtliche, erfindungsgemässe Katheter besteht im wesentlichen aus einem Arbeitskopf 1, einem damit fest verbundenen Schlauch 2 sowie einer Förderschraube 3, welche durch eine an sich bekannte, nicht dargestellte Antriebseinheit am proximalen Ende des Katheters in Rotation versetzbar ist. Ein Führungsdraht 4 durchsetzt den Katheter axial über seine gesamte Länge. Beim Einsatz des Katheters wird letzterer entlang des zuerst in das zu behandelnde Blutgefäss eingeführten Führungsdrahts 4 bis zur behandelnden Stelle vorgeschoben. Dieses Vorgehen erfolgt durch den Arzt meistens unter Röntgenkontrolle.

Der Arbeitskopf ist vorzugsweise in Richtung seines distalen Endes verjüngt ausgebildet. Dieses erleichtert das Einführen des Katheters in die teilweise verengten Blutgefässe und verhindert, dass es dabei zu inneren Verletzungen kommen kann.

Die Förderschraube 3 steht unter axialer Druck-Vorspannung und ist über ein Axial-Drucklager 5 auf dem Arbeitskopf 1 abgestützt. Diese Vorspannung verringert beim Rotieren der Förderschraube 3, unter hoher Drehzahl, (bspw. etwa 50'000 U/Min), in der Drehrichtung R die Vibrationen an der Förderschraube 3. Ausserdem ergibt diese Vorspannung beim Zusammenwirken der Förderschraube 3 mit dem Arbeitskopf 1 klar definierte Eingriffsverhältnisse. Beim Fördern des abgetragenen und zerkleinerten Materials in Richtung des proximalen Endes des Katheters entstehen zusätzliche Reaktionskräfte F, welche vom Axial-Drucklager 5 aufgenommen werden müssen.

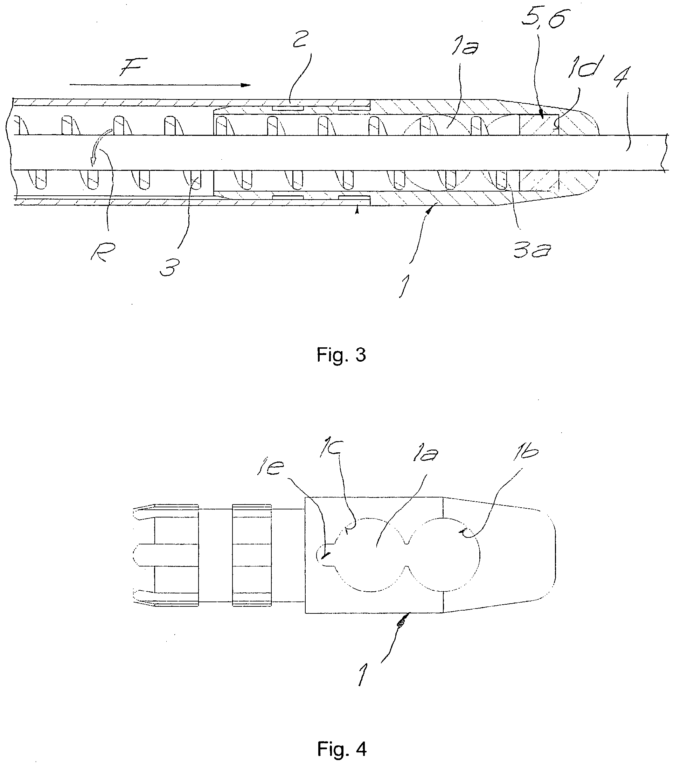

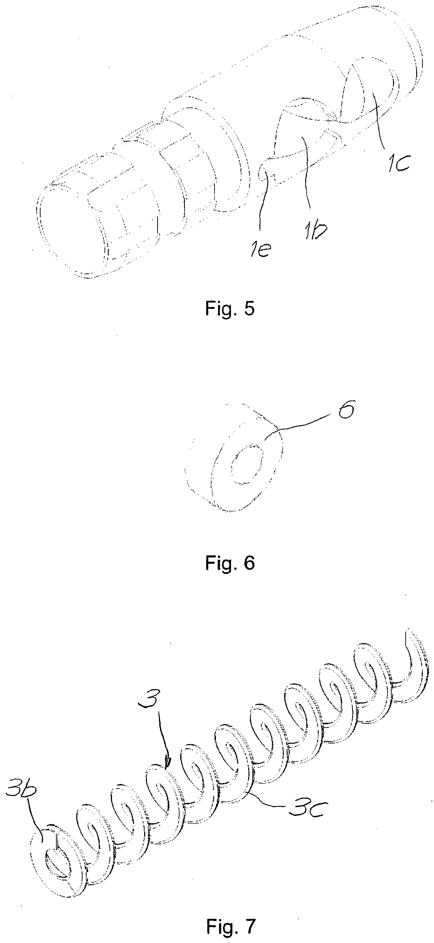

Wie aus dem Längsschnitt in Fig. 3 sowie der Fig. 6 deutlich hervorgeht, besteht das Axial-Drucklager 5 im wesentlichen aus einer gelochten Scheibe 6, welche zwischen dem distalen Ende der Förderschraube 3 und einer Schulter 1d des Arbeitskopfes 1 angeordnet ist. Die Scheibe 6 besteht aus einem harten und abriebfesten Kunststoff mit guten Gleiteigenschaften. Die Schmier- und Reibungsverhältnisse zwischen der Scheibe 6 und der Förderschraube 3 einerseits sowie der Scheibe 6 und dem Arbeitskopf 1 andererseits sind etwa gleich. Daher ist die durch Mitnahme erfolgende Relativverdrehung zwischen der Scheibe 6 und der rotierenden Förderschraube 3 etwa gleich, wie zwischen der Scheibe 6 und dem stillstehenden Arbeitskopf 1. Dieses bedeutet bzw. bewirkt, dass die Scheibe 6 beim Antrieb des Katheters etwa mit der halben Drehzahl der Förderschraube 3 mitdrehen wird. Dadurch wird der Verschleiss verringert und zudem die Wärmeentwicklung infolge von Reibung im Katheter reduziert.

Wie aus den Figuren 1, 2, 4 und 5 hervorgeht, weist der Arbeitskopf 1 seitliche Öffnungen 1 a auf. Diese Öffnungen 1 a sind beispielsweise als axial hintereinander angeordnete, kreisrunde Löcher 1 b, 1 c ausgebildet. Der axiale Abstand der beiden Löcher 1 b, 1 c ist vorzugsweise so ausgewählt, dass sich die Kreisumfänge der Löcher 1 b, 1 c überschneiden, d.h. der Achs-Abstand der Löcher 1 b, 1 c ist etwas kleiner als der Durchmesser der Löcher 1 b, 1 c. Am proximalen Ende der Öffnungen 1 a bzw. der Löcher 1 b, 1 c ist ein in axialer Richtung verlaufender Schlitz 1 e angeordnet. Sowohl die innenliegenden Kanten der Löcher 1 b, 1 c als auch diejenigen des Schlitzes 1 e sind vorzugsweise scharfkantig ausgebildet und bilden pro Öffnung 1 a insgesamt je fünf Schneidkanten 1f, 1g, 1h, 1i, 1k, wie aus Fig. 2 ersichtlich ist.

Um im Bereich der seitlichen Öffnungen 1 a günstige Strömungsverhältnisse zu erzielen, sind dagegen die Kanten der seitlichen Öffnungen 1 a im Bereich der Aussenmantelfläche des Arbeitskopfes 1 wenigstens bereichsweise als Abrundungen ("Verrundungen") 1l ausgebildet.

Die in Fig. 7 dargestellte Förderschraube 3 weist plane Endwindungen 3b auf. Am distalen Ende der Förderschraube 3 liegt diese Endwindung 3b auf der das Axial-Drucklager 5 bildenden Scheibe 6 auf. Die Kanten 3c an der Aussenseite der Förderschraube 3 sind wenigstens im Bereich des Arbeitskopfes 1 scharfkantig ausgebildet. Diese Kanten 3c bewirken zusammen mit den Schneidkanten 1 f, 1 g, 1 h, 1i und 1k des Arbeitskopfes 1 ein Zerschneiden, bzw. Zerkleinern des in den Katheter eingesaugten, abzutragenden Materials.

### Bezugszeichenliste

- 1: Arbeitskopf
- 1a: Öffnung
- 1b: Loch
- 1c: Loch
- 1d: Schulter
- 1e: Schlitz
- 1f: Schneidkante
- 1g: Schneidkante
- 1h: Schneidkante
- 1i: Schneidkante
- 1k: Schneidkante
- 1l: Abrundung ("Verrundung")
- 2: Schlauch
- 3: Förderschraube
- 3a: distales Ende
- 3b: Endwindung
- 3c: Kante
- 4: Führungsdraht
- 5: Axial-Drucklager
- 6: Scheibe
- F: Reaktionskraft
- R: Drehrichtung

## Patentansprüche

1. Katheter zum Ansaugen, Fragmentieren und Hinausfördern von entfernbarem Material aus Hohlkörpern, insbesondere von Thromben und Embolien aus Blutgefässen, mit einem über einen Führungsdraht (4) unabhängig von diesem axial verschiebbaren, an einem distalen Ende des Katheters angeordneten Arbeitskopf (1) mit einem Innendurchmesser einer Innenmantelfläche, welcher Arbeitskopf wenigstens eine seitliche Öffnung (1 a) aufweist, wobei der Katheter eine mittels eines vom Arbeitskopf (1) entfernten Drehantriebes einer Antriebseinheit mit einer Drehzahl in Rotation versetzbare flexible Förderschraube (3) mit einem Aussendurchmesser, distalen Ende (3a) und einem proximalen Ende aufweist, sowie mit einem die Förderschraube (3) umgebenden, mit dem Arbeitskopf (1) verbundenen flexiblen Schlauch (2) zur Abfuhr des Materials bzw. der abgelösten Thromben und Emboliefragmente und einem Schneidwerkzeug, wobei die Förderschraube (3) als mit der Öffnung (1 a) des Arbeitskopfes (1) zusammenwirkendes, scherendes Schneidwerkzeug ausgebildet ist, um zwischen den peripheren Rändern der Förderschraube (3) und Rändern der Öffnungen (1 a) die eindringenden Materialien bzw. angesaugten und/oder abgelösten Thromben und Embolien zu zerkleinern, **dadurch gekennzeichnet, dass** zwischen dem distalen Ende (3a) der Förderschraube und dem Arbeitskopf (1) ein Axial-Drucklager (5) angeordnet ist, das als eine beim Arbeitsgang durch die Förderschraube (3) in Rotation versetzbare Scheibe (6) ausgebildet ist.

2. Katheter nach Anspruch 1, **dadurch gekennzeichnet, dass** die Scheibe (6) aus einem Werkstoff mit geringem Reibungskoeffizienten, vorzugsweise aus Kunststoff, besteht.

3. Katheter nach einem der vorhergehenden Ansprüche, **dadurch gekennzeichnet, dass** die Förderschraube (3) unter axialem Druck vorspannbar ist.

4. Katheter nach Anspruch 3, **dadurch gekennzeichnet, dass** der Vorspanndruck der Förderschraube (3) einstellbar ist, wobei der Vorspanndruck der Förderschraube vorzugsweise so eingestellt ist, dass das Axial-Drucklager (5) etwa mit der halben Drehzahl der Förderschraube (3) mitrotiert.

5. Katheter nach einem der vorhergehenden Ansprüche, **dadurch gekennzeichnet, dass** die wenigstens eine seitlichen Öffnung (1 a) des Arbeitskopfes (1) als kreisrunde Löcher (1 b, 1 c) mit jeweils einem Kreisumfang ausgebildet ist.

6. Katheter nach Anspruch 5, **dadurch gekennzeichnet, dass** die wenigstens eine seitliche Öffnung (1 a) des Arbeitskopfes (1) als jeweils wenigstens zwei, axial hintereinander angeordnete Löcher (1 b, 1 c) ausgebildet ist.

7. Katheter nach Anspruch 6, **dadurch gekennzeichnet, dass** sich die Kreisumfänge der Löcher (1 b, 1 c) überschneiden.

8. Katheter nach Anspruch 6 oder 7, **dadurch gekennzeichnet, dass** die Löcher (1 b, 1 c) durch einen in axial Richtung verlaufenden, im wesentlichen zentrisch zu den Löchern (1 b, 1 c) angeordneten Schlitz (1 e) miteinander verbunden sind.

9. Katheter nach Anspruch 8, **dadurch gekennzeichnet, dass** sich der Schlitz (1 e) in Richtung zum proximalen Ende des Katheters über die Löcher (1 b, 1 c) hinaus erstreckt.

10. Katheter nach einem der vorhergehenden Ansprüche, **dadurch gekennzeichnet, dass** am Arbeitskopf (1) jeweils zwei einander etwa diametral gegenüberliegend angeordnete Öffnungen (1 a) angeordnet sind.

11. Katheter nach einem der vorhergehenden Ansprüche, **dadurch gekennzeichnet, dass** der distale Teil (3a) der Förderschraube (3) im Bereich des Arbeitskopfes (1) im Aussendurchmesser gegenüber dem Innendurchmesser des vorzugsweise zylindrischen Arbeitskopfes (1) passgenau ausgebildet ist, so dass der Aussendurchmesser der Förderschraube (3) zum Innendurchmesser der Innenmantelfläche des Arbeitskopfes (1) ein nur minimales Durchmesserspiel aufweist.

12. Katheter nach einem der vorhergehenden Ansprüche, **dadurch gekennzeichnet, dass** Kanten (3c) an der Aussenseite der Förderschraube (3) vorhanden sind, welche wenigstens im Bereich der Öffnung des Arbeitskopfes (1) scharfkantig ausgebildet sind.

13. Katheter nach einem der vorhergehenden Ansprüche, **dadurch gekennzeichnet, dass** der Arbeitskopf (1) in Richtung seines distalen Endes verjüngt ausgebildet ist.

14. Katheter nach einem der vorhergehenden Ansprüche, **dadurch gekennzeichnet, dass** die seitliche Öffnung (1 a) Schneidkanten (1 f, 1 g, 1 h, 1i, 1k) im Bereich der Innenmantelfläche des Arbeitskopfes (1) aufweist, welche wenigstens bereichsweise scharfkantig ausgebildet sind.

15. Katheter nach einem der vorhergehenden Ansprüche, **dadurch gekennzeichnet, dass** Kanten (1l) der seitlichen Öffnung (1 a) im Bereich der Aussenmantelfläche des Arbeitskopfes (1) wenigstens bereichsweise verrundet ausgebildet sind.

## Claims

1. Catheter for aspirating, fragmenting and removing removable material from hollow bodies, in particular thrombi and emboli from blood vessels, with a working head (1) with an internal diameter of an inner circumferential surface which is arranged on the distal end of the catheter and is displaceable axially along a guide wire (4) independently thereof, which working head (1) has at least one lateral opening (1a), wherein the catheter has a flexible feed screw (3) with an external diameter, a distal end (3a) and a proximal end which can be moved in rotation with a rotational speed by means of a rotary drive of a drive unit remote from the working head (1), and with a flexible hose (2) that surrounds the feed screw (3) and is connected to the working head (1) for removing the material and/or the detached thrombi and emboli fragments, and with a cutting tool, wherein the feed screw (3) is in the form of a shearing cutting tool that cooperates with the opening (1a) in the working head (1) to comminute the materials and aspirated and/or detached thrombi and emboli penetrating between the peripheral edges of the feed screw (3) and the edges of the openings (1a), **characterized in that** an axial thrust bearing (5) is arranged between the distal end (3a) of the feed screw and the working head (1), which bearing is designed as a disc (6) that can be caused to rotate by the feed screw (3) during the working cycle.

2. Catheter according to claim 1, **characterized in that** the disc (6) is made from a material having a low coefficient of friction, preferably plastic.

3. Catheter according to either of the preceding claims, **characterized in that** the feed screw (3) can be pretensioned with axial pressure.

4. Catheter according to claim 3, **characterized in that** the preload pressure of the feed screw (3) is adjustable, wherein the preload pressure of the feed screw is preferably set such that the axial thrust bearing (5) rotates with the feed screw (3) at about half the rotating speed thereof.

5. Catheter according to any one of the preceding claims, **characterized in that** the at least one lateral opening (1a) of the working head (1) is/are in the form of a circular holes (1b, 1c), each with its own circumference.

6. Catheter according to claim 5, **characterized in that** the at least one lateral opening (1a) of the working head (1) is/are designed as at least two holes (1b, 1c) arranged axially one behind the other.

7. Catheter according to claim 6, **characterized in that** the circumferences of the holes (1b, 1c) intersect with each other.

8. Catheter according to either of claims 6 or 7, **characterized in that** the holes (1b, 1c) are connected to each other via a slot (1e) that extends essentially centrally relative to the holes (1b, 1c) in the axial direction.

9. Catheter according to claim 8, **characterized in that** the slot (1e) extends beyond the holes (1b, 1c) towards the proximal end of the catheter.

10. Catheter according to any one of the preceding claims, **characterized in that** two openings (1a) are each arranged approximately diametrically opposite one another on the working head (1).

11. Catheter according to any one of the preceding claims, **characterized in that** in the area of the working head (1) the external diameter of the distal part (3a) of the feed screw (3) is designed to fit exactly inside the internal diameter of the preferably cylindrical working head (1), so that there is only minimal radial play between the external diameter of the feed screw (3) and the internal diameter of the inner circumferential surface of the working head (1).

12. Catheter according to any one of the preceding claims, **characterized in that** edges (3c) are present on the outside of the feed screw (3), which edges are designed with sharp edges at least in the area of the opening on the working head (1).

13. Catheter according to any one of the preceding claims, **characterized in that** the working head (1) is designed to taper towards the distal end thereof.

14. Catheter according to any one of the preceding claims, **characterized in that** the lateral opening (1a) has cutting edges (1f, 1g, 1h, 1i, 1k) in the area of the inner circumferential surface of the working head (1), at least parts of which are constructed with sharp edges.

15. Catheter according to any one of the preceding claims, **characterized in that** edges (11) at least parts of the lateral opening (1a) are rounded in the area of the outer circumferential surface of the working head (1).

## Revendications

1. Cathéter destiné à aspirer, à fragmenter et à extraire de la matière éliminable de corps creux, notamment de thrombus et d'embolies dans des vaisseaux sanguins, avec une tête de travail (1) déplaçable en direction axiale par l'intermédiaire d'un fil-guide (4) métallique, indépendamment de celui-ci, placée sur une extrémité distale du cathéter, avec un diamètre intérieur d'une surface d'enveloppe interne, laquelle tête de travail comporte au moins un orifice (1a) latéral, le cathéter comportant une vis de transport (3) flexible, susceptible d'être placée en rotation à une vitesse de rotation à l'aide d'un entraînement en rotation éloigné de la tête de travail (1) d'une unité d'entraînement, avec un diamètre extérieur, une extrémité distale (3a) et une extrémité proximale, ainsi qu'avec un tube flexible (2) entourant la vis de transport (3) relié avec la tête de travail (1) pour évacuer la matière ou les thrombus et fragments d'embolie décrochés et avec un outil de coupe, la vis de travail (3) étant conçue sous la forme d'un outil de coupe cisaillant, coopérant avec l'orifice (1a) de la tête de travail (1) pour fractionner entre les bords périphériques de la vis de transport (3) et le bords des orifices (1a) les matières pénétrantes ou les thrombus et embolies aspirés et/ou décrochés, **caractérisé en ce qu'**entre l'extrémité (3a) distale de la vis de transport et la tête de travail (1) est placé un palier de butée (5) axial qui est conçu sous la forme d'un disque (6) susceptible d'être mis en rotation par la vis de transport (3) lors de l'étape de travail.

2. Cathéter selon la revendication 1, **caractérisé en ce que** le disque (6) est constitué d'une matière à faible coefficient de friction, de préférence en matière plastique.

3. Cathéter selon l'une quelconque des revendications précédentes, **caractérisé en ce que** la vis de transport (3) est susceptible d'être précontrainte sous une pression axiale.

4. Cathéter selon la revendication 3, **caractérisé en ce que** la pression de précontrainte de la vis de transport (3) est réglable, la pression de précontrainte de la vis de transport étant réglée de préférence de sorte que le palier de butée (5) axial soit en co-rotation à environ la moitié de la vitesse de rotation de la vis de transport (3).

5. Cathéter selon l'une quelconque des revendications précédentes, **caractérisé en ce que** l'au moins un orifice (1a) latéral de la tête de travail (1) est conçu sous la forme de trous (1b, 1c) circulaires avec chacun une circonférence.

6. Cathéter selon la revendication 5, **caractérisé en ce que** l'au moins un orifice (1a) latéral de la tête de travail (1) est conçu chaque fois sous la forme d'au moins deux trous (1b, 1c placés l'un derrière l'autre en direction axiale.

7. Cathéter selon la revendication 6, **caractérisé en ce que** les circonférences des trous (1b, 1c) se recoupent.

8. Cathéter selon la revendication 6 ou la revendication 7, **caractérisé en ce que** les trous (1b, 1c) sont reliés ensemble par une encoche (le) s'écoulant en direction axiale, placée sensiblement de manière centrale par rapport aux trous (1b, 1c)

9. Cathéter selon la revendication 8, **caractérisé en ce que** l'encoche (le) s'étend en direction de l'extrémité proximale du cathéter, au-delà des trous (1b, 1c)

10. Cathéter selon l'une quelconque des revendications précédentes, **caractérisé en ce que** sur la tête de travail (1) sont placés chaque fois deux orifices (1a) mutuellement opposés de manière approximativement diamétrale.

11. Cathéter selon l'une quelconque des revendications précédentes, **caractérisé en ce que** dans la région de la tête de travail (1), par son diamètre extérieur, la partie (3a) distale de la vis de transport (3) est conçue en étant ajustée au diamètre intérieur de la tête de travail (1) de préférence cylindrique, de sorte que par rapport au diamètre intérieur de la surface d'enveloppe intérieure de la tête de travail (1), le diamètre vis de transport (3) ne présente qu'un jeu minimal au niveau du diamètre.

12. Cathéter selon l'une quelconque des revendications précédentes, **caractérisé en ce que** des arêtes (3c) qui au moins dans la région de l'orifice de la tête de travail (1) sont conçues à angles vifs sont présentes sur la face extérieure de la vis de transport (3).

13. Cathéter selon l'une quelconque des revendications précédentes, **caractérisé en ce que** la tête de travail (1) est conçue en étant rétrécie dans la direction de son extrémité distale.

14. Cathéter selon l'une quelconque des revendications précédentes, **caractérisé en ce que** dans la région de la surface d'enveloppe intérieure de la tête de travail (1), l'orifice (1a) latéral comporte des arêtes centrales (1f, 1g, 1h, 1i, 1k) qui au moins par régions sont conçues avec des angles vifs.

15. Cathéter selon l'une quelconque des revendications précédentes, **caractérisé en ce que** dans la région de la surface d'enveloppe extérieure de la tête de travail (1), des arêtes (11) de l'orifice (1a) latéral sont conçues en étant arrondies au moins par régions.
